Europäisches Patentamt

European Patent Office

Office européen des brevets

(11) Veröffentlichungsnummer: **0 346 595**
**A2**

## (12) EUROPÄISCHE PATENTANMELDUNG

(21) Anmeldenummer: **89107179.7**

(22) Anmeldetag: **21.04.89**

(51) Int. Cl.⁴: **A61K 6/04**

(30) Priorität: **11.06.88 DE 3819907**

(43) Veröffentlichungstag der Anmeldung:
**20.12.89 Patentblatt 89/51**

(84) Benannte Vertragsstaaten:
**AT BE CH DE ES FR GB GR IT LI LU NL SE**

(71) Anmelder: **Degussa Aktiengesellschaft**
**Weissfrauenstrasse 9**
**D-6000 Frankfurt am Main 1(DE)**

(72) Erfinder: **Schiwiora, Harry**
**Theodor-Heuss-Strasse 37**
**D-7530 Pforzheim(DE)**
Erfinder: **Stümke, Manfred, Dr. Dipl.-Phys.**
**Am Waldweg 21**
**D-7530 Pforzheim(DE)**
Erfinder: **Wagner, Rudolf, Dr. Dipl.-Ing.**
**Breslauer Strasse 10**
**D-7537 Remchingen 3(DE)**

(54) **Verwendung einer nichtedelmetallfreien Dentallegierung für gegossenen festsitzenden Zahnersatz.**

(57) Nichtedelmetallfreie Dentallegierungen mit guthaftender Oberfläche für Keramikverblendungen und geringer Verfärbungsneigung enthalten 60 bis 85 Gew % Gold, 4 bis 20 Gew % Platin, 2 bis 20 Gew % Palladium, 1 bis 11 Gew % Silber und 0,1 bis 3 Gew % Iridium und/oder Rhodium.

EP 0 346 595 A2

# Verwendung einer nichtedelmetallfreien Dentallegierung für gegossenen festsitzenden Zahnersatz

Die Erfindung betrifft die Verwendung einer nichtedelmetallfreien Dentallegierung auf der Basis von Gold zur Herstellung von gegossenem festsitzenden Zahnersatz. Dieser Zahnersatz kann sowohl unverblendet bleiben als auch mit Kunststoff oder mit keramischen Massen verblendet werden.

Üblicherweise werden Edelmetallegierungen für die konventionelle Kronen- und Brückentechnik mit in bezug auf Anteil und Art unterschiedlichen Nichtedelmetall-Zusätzen versehen, um Eigenschaften der Legierungen, wie z. B. Härte, Festigkeit, Dehnung, Schmelzintervall oder Aufbrennverhalten zu modifizieren. Solche Zusätze werden beispielsweise in der DE-PS 1533233 beschrieben.

Wie in der DE - OS 34 47 413 ausgeführt, besteht bei nichtedelmetallhaltigen Legierungen die Gefahr, daß bei dentalen Güssen mit solchen Legierungen durch Inhomogenitäten, Mikroporositäten und Oxidationsvorgängen eine Anreicherung der Nichtedelmetall-Bestandteile an der Legierungsoberfläche auftritt. Die so an der Oberfläche angereicherten Elemente rufen bei verschiedenen Patienten mitunter allergische Reaktionen hervor.

Es besteht daher der Wunsch nach nichtedelmetallfreien Legierungen, bei denen keine Segregationen von Nichtedelmetall-Legierungsbestandteilen auftreten können. Solche Legierungen bestehen im allgemeinen ausschließlich aus Gold mit Zusätzen von Palladium, Platin und Iridium (DE-OS 3447413).

Durch das Fehlen von Nichtedelmetallen bilden diese Legierungen keine haftvermittelnde Schichten aus und weisen daher nur eine schlechte Haftung der Aufbrennkeramik auf dem Metallgerüst auf.

Edelmetallegierungen, die größere Mengen Silber enthalten, verleihen in vielen Fällen beim Aufbrennen von Dentalkeramiken diesen eine unerwünschte grünliche Färbung.

Es war daher Aufgabe der vorliegenden Erfindung eine nichtedelmetallfreie Dentallegierung auf der Basis von Gold zur Herstellung von gegossenem festsitzendem Zahnersatz zu entwickeln, die auf der Oberfläche haftvermittelnde Schichten für die Verblendung mit Dentalkeramiken ausbildet und nicht zu Verfärbungen der Keramikschichten neigt.

Diese Aufgabe wird durch die Verwendung von Legierungen gelöst, die 60 bis 85 Gew % Gold, 4 bis 20 Gew % Platin, 2 bis 20 Gew % Palladium, 1 bis 11 Gew % Silber und 0,1 bis 3 Gew % Iridium und/oder Rhodium enthält.

Vorzugsweise verwendet man Legierungen mit 70 bis 85 Gew % Gold, 5 bis 12 Gew % Platin, 3 bis 15 Gew % Palladium, 2 bis 6 Gew % Silber und 1 bis 2,5 Gew % Iridium und Rhodium.

Überraschenderweise hat es sich gezeigt, daß bei nichtedelmetallfreien Legierungen von Gold und Platin und Palladium sowie Iridium oder Rhodium, die zwecks Kornfeinung zulegiert werden, durch den Zusatz von Silber auch ohne Verwendung von haftvermittelnden Zwischenschichten eine sehr gute Haftung der Keramik auf dem Metallgerüst erreicht werden kann. Weiterhin stellte sich überraschenderweise heraus, daß diese Legierungen, obwohl sie bis zu 11 % Silber enthalten können, verfärbungempfindliche Keramiken nicht grünlich verfärben. Dies ist umso überraschender, da von Legierungen auf Palladium-Basis bekannt ist, daß bereits geringe Zusätze an Silber bei manchen Aufbrennkeramiken starke Verfärbungen in Bereichen geringer Keramikschichtstärke hervorrufen. Der Zusatz von Silber bietet zudem die Möglichkeit, trotz höherer Palladium- und Platinzusätze, die zur Erreichung einer ausreichend hohen Festigkeit der Legierungen unerläßlich sind, das Schmelzintervall dieser Legierungen so niedrig halten, so daß diese mit den im zahntechnischen Labor bisher üblichen Geräten ohne Probleme erschmolzen und vergossen werden können. Die Härtewerte dieser Legierungen liegen oberhalb von 75 HV 5/30. Zudem wirken Silberzusätze in bezug auf das Wärmeausdehnungsverhalten insofern regulierend, als der durch Palladium- und Platin-Zusätze abgesenkte Wert des Wärmeausdehnungskoeffizienten dieser Legierungen durch Silberzusätze wieder auf Werte angehoben werden kann, die eine rißfreie Verblendung mit keramischen Massen zulassen. Legierungen mit Silber-Anteilen 11 % weisen hingegen unzulässig hohe Werte des Wärmeausdehnungskoeffizienten auf und beginnen bei verschiedenen Verblendkeramiken grünliche Verfärbungen zu verursachen.

Die Tabelle zeigt die Zusammensetzung einiger beispielhafter Legierungen mit ihren Härtewerten nach dem Guß, der Beurteilung ihrer Haftung an Dentalkeramiken und ihrer Verfärbungsneigung. Diese Legierungen sind für gegossenen festsitzenden Zahnersatz sehr gut geeignet.

Tabelle

| Legierungszusammensetzung | | | | | | Härte HV nach dem Guß | Farbneutralität auch bei verfärbungsempfindlichen Keramiken | Haftung der Keramik auf dem Brückengerüst |
|------|------|------|-----|------|------|------|------|------|
| Au | Pt | Pd | Rh | Ir | Ag | | | |
| 85,0 | 5,0 | 3,25 | 1,6 | 0,15 | 5,0 | 80 | + + | + + |
| 80,0 | 11,0 | 4,2 | 1,6 | 0,2 | 3,0 | 95 | + + | + + |
| 70,0 | 7,5 | 15,0 | 2,0 | 0,5 | 5,0 | 130 | + + | + + |
| 65,0 | 7,5 | 15,0 | 2,0 | 0,5 | 10,0 | 105 | + | + |
| 66,0 | 7,5 | 20,0 | 2,0 | 0,5 | 4,0 | 115 | + + | + |
| 68,0 | 4,0 | 19,0 | 1,6 | 0,4 | 7,0 | 105 | + | + |
| 68,5 | 7,5 | 17,0 | 1,6 | 0,4 | 5,0 | 110 | + + | + |
| 68,5 | 20,0 | 4,0 | 2,0 | 0,5 | 5,0 | 90 | + + | + |
| 70,0 | 4,6 | 13,0 | 2,0 | 0,4 | 10,0 | 90 | + | + |
| 70,0 | 12,0 | 6,4 | 1,6 | - | 10,0 | 80 | + | + + |
| 72,5 | 12,0 | 6,5 | 1,6 | 0,4 | 7,0 | 80 | + | + |
| 81,0 | 8,0 | 6,0 | 2,0 | 0,5 | 2,2 | 80 | + + | + |

Bewertungsschlüssel:

+ gut

+ + sehr gut

## Ansprüche

1. Verwendung einer nichtedelmetallfreien Dentallegierung auf der Basis von Gold mit 60 bis 85 Gew % Gold, 4 bis 20 Gew % Platin, 2 bis 20 Gew % Palladium, 1 bis 11 Gew % Silber und 0,1 bis 3 Gew % Iridium und/oder Rhodium für gegossenen festsitzenden Zahnersatz.

2. Verwendung einer Legierung mit 70 bis 85 Gew % Gold, 5 bis 12 Gew % Platin, 3 bis 15 Gew % Palladium, 2 bis 6 Gew % Silber und 1 bis 2,5 Gew % Iridium und Rhodium gemäß Anspruch 1.